# EUROPEAN PATENT APPLICATION

(11) **EP 4 813 363 A1**
(43) Date of publication of application: **30.09.2026**
(21) Application number: 25166895.0
(22) Date of filing: 28.03.2025
(51) Int. Cl.: A61F 13/15, D04H 1/425, D04H 1/732, D04H 1/736

(54) **INLINE INSPECTION AND CONTROL SYSTEM FOR AIR-LAID WEBS**

(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention is a contact free control system employing electromagnetic wave analysis in the manufacturing of fibrous webs, such as may be suitable for being used for absorbent articles, such as baby diapers, adult incontinence articles, but especially bed pads, meat pads or the like. In particular, the present invention relates to a control system in the manufacturing of air-laid webs, which can be directly fed into further article manufacturing converter lines.

## Description

### Field of the Invention

The present invention relates to a contact free control system employing electromagnetic wave analysis in the manufacturing of fibrous webs such as may be suitable for being used for absorbent articles, such as baby diapers, adult incontinence articles, but especially bed pads, meat pads or the like. In particular, the present invention relates to a control system in the manufacturing of air-laid webs, which can be directly fed into further article manufacturing converter lines.

### Background of the invention

Contact free control systems in the manufacture of absorbent articles are well known in the art, see e.g., EP2726039, wherein the positioning of various elements of an article are controlled by an image control system.

However, such a system can only view the outer contours of the article, such as fastening tapes. There is still a need for an in-line control system that also analyses "inner" parameter of the articles at a high speed, especially of the absorbent system, and more particular the amount and distribution of superabsorbent polymers (SAP).

WO2019229273A1 (CCSol) describes a manufacturing system for absorbent structures with a detection system to quantitatively determine and control at high speeds the amount of superabsorbent polymeric (SAP) particles in an SAP/fluff mix by employing a video analysis system using radio frequency analysis, as generally known from WO2018/039535 (Accusentry) or WO2018/204724 (Alcatera) determining the amount of SAP and fluff in the analyzed sample. In EP3943917B1 (Fameccanica), a method for the in-line analysis of a composite product (10) with SAP and fluff is described, wherein a hyperspectral sensor is used to acquire images of samples of target materials that are part of the composite product, in order to perform an in-line optical inspection at process speed. Other image analysis systems are available on a commercial basis, such as from Cognex Corp., MA, USA, or Mitsubishi Electric Corp., JP.

However, there is still a need for setting up a simple manufacturing system for the accurate and effective production of fibrous webs in particular when made by an air-lying process. It is an object of the present invention to dry lay, or air-lay, such fibrous material to form fibrous webs for being converted into articles comprising such fibrous webs.

### Summary of the Invention

In a first aspect, the present invention is an equipment for forming an air-laid web comprising cellulose fibers. The equipment is comprising
1) a short fiber supply unit for supplying fibrous material comprising cellulosic fibers,
   - adapted to provide varying fiber feed rates;
2) a fiber individualizing unit, preferably fiber pick apparatus or a hammer mill,
   - adapted to operate at varying individualization degrees;
3) a fiber-in-air suspension unit comprising
   3a) a suspension air supply, adapted to adjust
      - temperature, water content, air flow rate;
   3b) a mixing unit, preferably a venturi unit,
      - adapted to suspense the fibrous material in the air;
4) a web forming unit comprising;
   4a) a forming box;
   4b) a homogenization unit comprising a multiplicity of impellers, rotatably mounted around z-directional axis, and comprising
   4b1) a speed adjustable drive for one or more of the impellers;
   4b2) blades, preferably with adjustable blade angles;
5) a fiber collection unit, comprising
   5a) an air permeable collector, preferably a foraminous belt, operating at an adjustable collector speed, adapted for deposition of the fibrous material thereon, thereby forming a web;
   5b) a vacuum suction unit, comprising
   5b1) an adjustable air blower adapted to be operated at varying air vacuum and volume;
   5b2) a multiplicity of vacuum suction boxes connected to the air blower, preferably comprising air flow adjustment baffles;
   5b3) optionally a web compaction unit;
   5b4) optionally a reject system;
6) a contact free inspection unit
   6a) comprising at least one, preferably a multiplicity of receiver units,
   6ai) arranged
      - moveable at least cross-directionally or
      - in a line or a 2D-matrix;
   6aii) adapted to receive electromagnetic (EM) waves from a surface of a web formed on the collector
   6aiii) and adapted to convert EM waves in the range of from 10 Hz to 1019Hz, preferably in a range selected from the group consisting of
      - visible light of about 10¹⁴ to 10¹⁵ Hz
      - 10¹¹ to 10¹² Hz;
      - 3x10³ Hz to 3x10⁹ Hz
      into an electric signal;
   6b) optionally at least one emitter unit providing electromagnetic waves with a frequency in the range of from 10 Hz to 10¹⁹Hz to be received by the receiver unit(s);
7) a data processing unit adapted
   - to convert the electric signal of 6a) into an at least x-y-directionally expanding actual property image
      preferably with an image resolution of the smaller of at least three times the size of a receiver unit and resolution size that is less than about 5 mm by 5 mm, preferably better than 1 mm by 1 mm, more preferably less than 0.3 mm by 0.3 mm or most preferably less than 0.1 by 0.1 mm.
   - and to compare the actual property image to a target property image and a deviation image,
   - and to calculate signals for adjusting at least one of the processing parameters of at least one of the units 1) to 5),
      thereby calculating the occurrence of nits, clumps, and spots by changes in the deviation image of more than 30 % of the signal, whereby
         nits exhibit a size of equal or less than about 4 mm²;
         clumps exhibit a size of more than about 4 mm² and less than about 64 mm²;
         spots exhibit a size of more than about 64 mm²;
      and wherein each of the units 1 to 5) are adjustable to impact at least one of the web property parameters selected from the group of
   - nits occurrence;
   - clump occurrence;
   - spots occurrence;
   - a basis weight.

The EM waves may exhibit a wave frequency in the range of visible light of 10¹⁴ to 10¹⁵ Hz. The contact free inspection unit and EM wave emitter may be positioned at the same side relative to the web.

In a second aspect, the present invention is a process for the manufacture of air-laid webs as may be operated on such an equipment and further comprises the steps of
b) operating the equipment by
b1) creating a fiber-in-air suspension by individualizing fibers from the fiber supply unit in an air stream;
b2) feeding the fibers into the web forming unit and forming a web on the collector of the fiber collection unit operating at a collector speed;
c) inspecting the web by the inspection unit by receiving electromagnetic waves by the receiver unit(s), the inspecting comprising the steps of receiving a time depending signal
as a function of the EM wave response parameter as a function of the web property parameters by the receiver unit(s), and transmitting the signal to the data processing unit;
d) analysing the signal in the data processing unit by
   - creating a parameter image of the web properties, showing a time dependent CD profile of the web property parameters;
   - comparing the parameter image to a pre-determined set of target parameters;
   - determining local and time dependent variations of the parameters of the web by synchronizing the parameter image with the collector speed;
   whereby steps b) to f) are executed continuously.
e) providing correction signals for any of the units 1) to 5) of the equipment,
   by comparing the local and time dependent variations of the parameters to a predetermined set of adjustment parameters;
f) adjusting processing parameters of the units 1 to 5),

Such a process may provide a web with a basis weight of more than about 10 g/m², or less than about 200 g/m².

The EM waves received by the receiver unit(s) may be in a range selected from the group consisting of
- visible light of about 10¹⁴ to 10¹⁵ Hz;
- 10¹¹ to 10¹² Hz;
- 3x10³ Hz to 3x10⁹ Hz.

In yet a further aspect, the present invention is a computer implemented control method in a manufacturing system for fibrous webs comprising the steps of
A) providing a data processing unit comprising a data analysis algorithm, a target data storage and a deviation data storage, further a human interface device and an output data storage device;
B) receiving in the data processing unit
   a. electrical signals from an EM detection system and
   b. speed related trigger signals from the collector drive controller;
C) generating an actual cross-directional deviation profile by comparing the signals from the EM detection system and the target data storage data in the data processing system for defined regions of the web;
D) comparing the actual deviation profile to the deviation data storage data and calculating reaction parameters by predetermined algorithms;
E) sending the reaction parameter of step D) to one or more of
   a) control of the fiber supply by control unit for
      a1) fiber board supply drive and/or
      a2) fiber bale picker unit;
   b) control of the disintegration unit, such as a hammer mill, through disintegration rotational speed controller 1214 for the rotor drive 1213 and/or disintegration gap width controller 1217 by disintegration gap width 1216;
   c) control of the transfer air supply, especially
      c1) temperature control;
      c2) humidity control;
      c3) air flow volume control;
   d) control of the web forming unit, especially for each individual or a group of impellers by
      d1) control of rotational speed, and/or
      d2) control of the angle of the impeller blades;
   e) control of the collector unit, especially
      e1) control of the machine directional speed of the air permeable collector;
      e2) control of main suction fan;
      e3) control of suction for each individual or a group of vacuum suction boxes;
   f) control of the reject actuator;
F) actuating the respective controller unit according to step E,
whereby steps B to F are essentially continuously executed.

### Brief description of the drawings

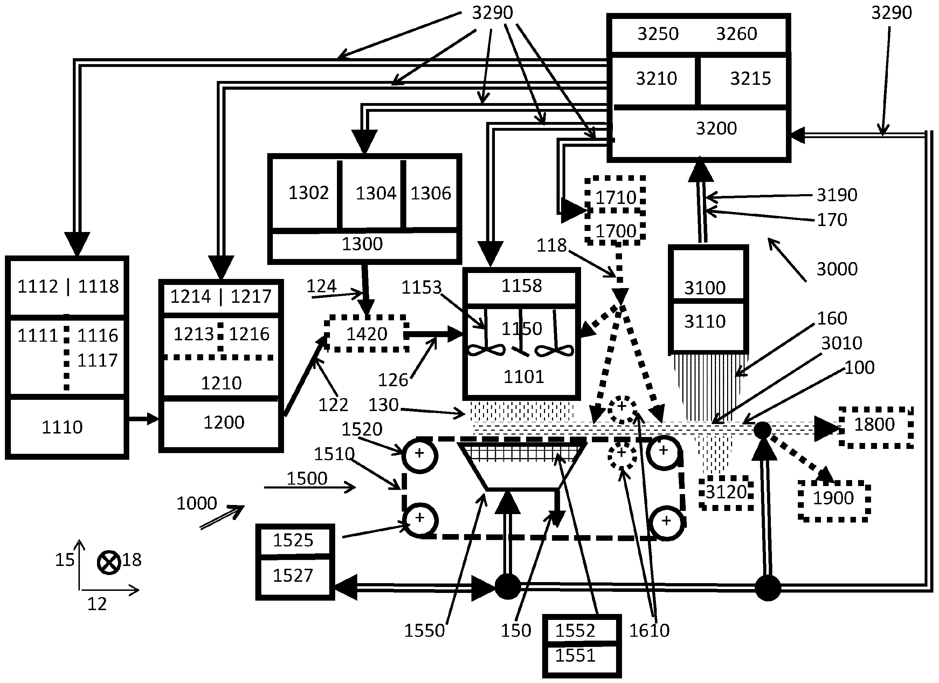

Fig. 1 depicts an equipment according to the present invention for operating a process according to the present invention.

The figures are schematic only, and not to scale. Same numerals refer to same or equivalent features or elements, single (') or multiple (", ‴, ...) apostrophes indicate duplicate features, such a left and right or front and back, etc..

### Detailed Description

In a first aspect, the present invention is a continuously operated equipment for the manufacture of fibrous webs comprising short fibers, preferably cellulosic fibers such as wood pulp fibers, as may be used in further processing steps to produce consumer goods such as absorbent pads as may be used for bed pads, meat pads, or animal pads, or wipes. In another aspect, the present invention is a process for manufacturing such webs, whereby the process preferably feeds into downstream converting equipment for finishing such articles, such as combining such webs with further fibrous webs or adding particulate material, such as superabsorbent polymers (SAP).

In yet another aspect, the present invention is computer implemented method of controlling a manufacturing process for such absorbent webs.

One type of preferred fibers for the fibrous web comprises natural fibers including wood pulp fibers as may be formed by a variety of pulping processes, such as kraft pulp, sulphite pulp, thermomechanical pulp, and the like. Further, the wood fibers may be any high-average fiber length wood pulp, low-average fiber length wood pulp, or mixtures of the same. One example of suitable high-average length wood pulp fibers include softwood fibers such as, but not limited to, northern softwood, southern softwood, redwood, red cedar, hemlock, pine (e.g., southern pines), spruce (e.g., black spruce), combinations thereof, and the like. One example of suitable low-average length wood pulp fibers includes hardwood fibers, such as, but not limited to, eucalyptus, maple, birch, aspen, and the like. The fibers may be treated so as to allow optimization of processing and or absorbency properties, but not necessarily need to be. The term "treated" as used herein is understood to include any means of introducing the additive to the fiber and/or fibrous matrix, but not limited to, such as coating, spraying, printing, chemical modifications, wet-end additions applications to the fibers as well as blending untreated fibers with treated fibers. As "treated" fibers typically incur a cost upcharge, it may be preferred to use untreated fibers. Moreover, if desired, secondary fibers obtained from recycled materials may be used, such as fiber pulp from sources such as, for example, newsprint, reclaimed paperboard, and office waste, or recycled diapers, be it from factory scrap or be it post-consumer recycling. The web may further comprise short man-made fibers, as may be made from synthetic polymeric, typically thermoplastic material, such as polyolefins, especially polypropylene or polyethylene, or polyesters, or from modified natural materials, such as viscose / rayon, cellulose acetate, or polylactate, polybutyrate, polyvinyl-acetate or - alcohol and the like. Diameters may range from less than about 0.001 mm to more than about 0.2 mm and they come in several different forms such as short fibers (known as staple, or chopped). Such man-made fibers could be added intentionally, so as to allow bonding at a later processing step, or unintentionally, such as when recycled or reclaimed material are used, but should not be present in more than about 20 weight-%, or not more than about 10 weight-%, or not more than about 5 weight-% of the web.

The fibrous material may be delivered as fibrous board or in fibrous sheet form, and typically is disintegrated in a disintegrator, such as a hammer mill. Also bales of fibrous material may be delivered as may be opened, e.g., by a picker roll system. Further, "roughly graded material" may be employed, wherein fibers are present as clusters of several hundred up to several thousand fibers in the roughly graded material.

Within the present context, a "web" is a manufactured sheet or batt of directionally or randomly orientated fibers, initially unbonded, but later in the process it may be bonded by friction, and/or cohesion and/or adhesion, excluding wet-laid paper and products which are woven, knitted, tufted, stitch-bonded incorporating binding yarns or filaments, or felted by wet-milling, whether or not additionally needled. The basis weight of a web is usually expressed in grams per square meter (g/m² or gsm). A web made on an equipment or by a process according to the present invention may be transferred to further processing equipment or steps for the manufacturing of articles, such as - without limitation - absorbent articles such as wipes, bed-pads, diapers, feminine hygiene or incontinence articles.

Optionally, the web may be combined with superabsorbent polymers, hereinafter also referred to as "SAP", such as well-known to a person skilled in the art of disposable absorbent articles, e.g., by intermixing the SAP with fibers during deposition, or by sprinkling SAP particles onto a formed web, or by embedding SAP between layers of such webs.

Articles made with fibrous webs according to the present invention may exhibit a wide range of dimensions such as an article width that may be as low as 1 cm or even less, or less than about 10 cm or less than about 20 cm, or less than about 30 cm, or less than about 50 cm. For even larger articles, such as bed pads, the width may be as wide as 100 cm or even more. Absorbent structures or cores of such articles are typically slightly smaller than the articles, such that there may be an absorbent free perimeter around the absorbent core to enable sealing that may be as small as 5 mm, often be around 10 mm, but reaching up to 50 mm, whereby the perimeter width may vary around the article.

Often, though not necessarily, the length of the article exceeds the width thereof. The length may be as low as 10 cm or even less, or less than about 20 cm, or less than about 30 cm, or less than about 50 cm. For even larger articles, such as bed pads, the length may be as long as 100 cm or even more.

Within the present context, a fibrous web exhibits predetermined "target" properties that may be the same for the total web or for predetermined sub-regions of the web. The parameter for describing the properties may be expressed by an average for the web or a sub-region thereof. Alternatively, the parameter may be expressed as a "local" value, referring to a value determined by measurements for smaller area units, such as of a 5 mm by 5 mm square, or the size of a "pixel", referring to the size of a receiver cell and the respective resolution, as will be discussed in more detail herein below.

Particularly important properties of a fibrous web are as follows:
- Basis weight as expressed in g/cm², as may be determined for homogeneous structures be weighing, such as of cut samples. For smaller areas, or x-y-directionally inhomogeneous basis weight pattern, detectors as discussed hereinbelow provide a correlating signal, often a proportional signal, such as a shade of grey tones for visual light. Too high basis weight variations are not desired from a performance, material usage, and appearance point of view, and preferably the variation for a given area should be less than about 25%, or less than about 10% or even less than about 5%.
- Fiber aggregates, as nits, clumps, or spots, in contrast to "individualized" fibers. The latter refers to fibers, which have no contact to neighbouring fibers (such as when being suspended in an air stream) or - when such fibers are laid down from such an air stream onto the surface of a collecting device - for which there are essentially no bonding forces. Such bonding forces may be desired at a later stage in the manufacturing process, and may be created by compressing the web or by adding bonding agents, but at the formation stage of the web such bonding is undesired to achieve a homogeneous web structure, at least at a predetermined local region.

Nits are small tangled fiber bundles of typically about or less than about 4 mm² in size, which may decrease the quality of the non-woven web, primarily by deteriorating visual appearance. Often, up to about 100 nits/m² are considered acceptable, though it is more preferred to have less than about 50 nits/m², or less than about 10 nits/m² or even less than about 5 nits/ m². The occurrence of nits may be controlled by controlling especially the properties of the transport air of fibers, such as water content.

Fiber aggregates of more than about 4 mm², but less than about 64 mm² are referred to as clumps, as may result from improper temperature management. As the clumps exist in a very compact form, these may deteriorate not only the visual appearance but also other quality parameters, such as absorbency. Whilst this might be compensated by increasing the supply of fibers by a quantity of fibers corresponding approximately to the mass of fibers tied up in the clumps, thereby increasing the cost of producing the web, other control mechanisms are preferred, like controlling the air flow, and especially the air temperature, for the transfer air during and after the disintegration step. Often, up to about 50 clumps/m² are acceptable, though it is more preferred to have less than about 20 clumps/m², or less than about 5 clumps/m², or even less than about 3 clumps / m².

Together, nits and clumps may make up for about 2 weight-% in the formed web, but preferably are less the about 1.4 w-%, or less than about 1.0 w-%. It should be noted that the proper control of nits and clumps is even more important when the above described "untreated" pulp is used, as the treatment additives are - among other purposes - directed towards reducing formation of such aggregates.

Fiber aggregates of more than about 64 mm² are considered "spots" and are not desired to remain in the product to which the web is converted. Henceforth, the region of the web should be rejected, either as a portion of the web or as a defect product after converting the web.

The fiber supply, such as the fiber board, sheet, or bale, contains some nits already from the start. During the defibration process some of these fiber aggregates are opened and formed into good individualized fibers, typically suspended in an air stream. But at the same time some other fibers are formed into nits in a normally larger scale than the quantity of fibers tied up in nits, which are opened.

Typically, the fibrous raw material is delivered in the form of a compressed fibrous board or bales and a disintegrating tool, such as a hammer mill, or a pick-up tool, such as a fiber picker, break up the fiber bonds. However, such disintegration may also result in undesired fiber break up, if the disintegration is too harsh, as may result from a change in the raw material properties.

However, even well disintegrated and individualized fibers may form aggregates before they are deposited onto a collection unit. Such re-aggregation may occur if the fibers are suspended in the air stream after the disintegration unit due to various reasons, especially due to electrostatic effects or aerodynamics, such as vortices.

In order to prevent re-aggregation of fibers forming nits or clumps, it is preferred that the air in which the fibers are suspended, is well controlled for the following parameters:
- Air temperature and humidity to reduce electrostatic effects without "over-moisturization" that may increase stickiness or even affect absorbency in the resulting article.
- Air flow rate, but also "smoothness" to avoid vortices or dead spaces such that the fiber-to-fiber contacts or collisions with the apparatus walls are minimized during the fiber transport and transfer.

The x-y-directional distribution of the fibers during lay down, also referred to a homogeneity, may be enhanced by feeding the disintegrated fibers into a forming box comprising a multiplicity, typically an array, of rotating impeller, which may reduce, but under sub-optimal air conditions, also increase re-agglomeration of the fibers.

Thus, in order to ensure quality evenness across time and length and width of the fibrous web, the present invention provides a system for forming an air laid web, which is further adapted to
- automatically measuring and analysing properties of the formed web,
- compare the actual properties to a pre-set pattern of properties as well as to a pre-set deviation pattern of these properties, and
- adjust automatically process parameter according to a pre-set process parameter adjustment algorithm.

In order to explain the aspects of the present invention, reference is made to Fig. 1 exhibiting the principles of a conventional air-laying equipment 1000 on which the process for forming a fibrous web 100 can be executed, exhibiting a machine (x-) direction (12), a cross (y-)direction (18) and a height (z-)direction (15). further including the measurement, analysis, and adjustment tools according to the present invention.

Along a process path, fibers are delivered by a fiber supply unit 1110, comprising a fiber supply controller 1112 for adjusting the amount of delivered fibers, e.g., adjusting the drive speed of a fiber board supply roll 1111 and/or controlling 1118 a bale opening picker device 1116 of a bale 1117. Optionally, the fiber delivery may comprise a first and a second fiber supply unit, for example for delivering rolls or virgin pulp board and recycled fibers from other process steps, such as in bale form or from a recycled fiber storage bin, with all further fiber supply units comprise their own control system.

The fibers are transferred to a fiber individualizing unit 1200, aiming at "individualizing" the delivered fibers, i.e., breaking up most, preferably all, of the fiber-to-fiber bonds and comprising a controller 1214 for adjusting the intensity of the individualizing unit, whilst avoiding or at least minimizing fiber break up. In a preferred execution, the disintegration unit 1200 comprises a hammer mill 1210, driven by a drive 1213 controlled by a drive controller 1214 and exhibiting an adjustable gap width 1216 between counterrotating rolls as may be adjusted by a gap width controller 1217, as such well known in the art. In the present context, and in contrast to many conventional arrangements where the fibers are fed cross-directionally, it is preferred that the rotating axis of the disintegrator is not machine-directionally, but more preferably cross-directionally oriented, thus providing the benefit that the fibers do not need to change the direction of flow but are already on a machine directional path, easing the suspension and homogenization in the transporting air. The disintegrated fibers 122 are further transferred to a forming box 1101 by means of transfer air 124 from an air supply 1300, forming a fiber-in-air-suspension 126 The properties of the transfer air can be controlled at least for temperature 1302, humidity 1304, and flow volume 1306. In a preferred execution, the fibers and the transfer air are combined in a venturi unit 1420, in Fig. 1 indicated as optional by dotted frame. The forming box 1101 comprises a fiber homogenizer system 1150 which allows to homogenize the distribution of the fibers in the x-direction (12) as well as in the y-direction (18), as may be a multiplicity of ventilator impeller 1153.

In Fig. 1 there are exemplarily but not limiting shown three impeller 1153 each with two impeller blades, rotating by means of drives around vertical axes at a rotating speed that can be controlled for each of the impellers individually or by groups of impellers by impeller controller 1158, by which also blade angle may be adjusted. As indicated in the cross-sectional view of Fig.1 three such impellers 1153 are schematically shown in the forming head 1101, but - as indicated in the background section - there could be an array of impellers, with two, three, four or even more impellers arranged in the cross-directional rows and three, or four, or five, or six or even more arranged in machine-directional columns. Optionally, the impellers in adjacent rows may be cross-directionally off-set, or the rows and columns may be in an angled position relative to the machine direction.

The individualized and x-y-directionally evenly distributed fibers 130 are then deposited though the forming box outlet onto the surface of a foraminous collection belt 1510 of the collection system 1500 moving along the machine direction 12 via collection system guide rolls 1520 and collector belt drive 1525 with a control unit 1527, which - as will be discussed in more detail herein below - is adapted to measures the collector speed to allow time synchronization of the other control unit and to adjust the speed as necessary, thereby defining the machine directional (12) speed of the collection belt 1510 and the web 100 deposited thereon.

The deposition of the fibers 130 is aided by a vacuum suction system 1550 positioned opposite of the forming box 1101 relative to the collection belt 1510. The vacuum suction system 1550 comprises a multiplicity, preferably an array, of suction chambers 1552 that can be individually adjusted for a varying degree of suction. Preferably, these suction chambers are connected to a single vacuum source drawing the combined suction air 150 with adjustable air flow controller 1551, such as valves or flaps, for each or a group of the suction system chambers 1552, though multiple vacuum sources can be appropriately connected.

Optionally (and thus being indicated with dotted lines in Fig. 1), and often preferably for ease of processing, a web compaction unit 1610, such as calender or compression rolls, reduces the thickness of the freshly formed web, thereby also increasing density and web integrity.

For certain uses, it may be preferred to add particulate material to the web. A particular application is when the web is further converted into absorbent articles, such as diapers, or pads, such as bed pads. To this end, an optional particle application system 1700 may be included, comprising a particle applicator control, unit 1710. The particles 118 may be added before or after the formed web is compacted in the (optional) compaction unit 1610, or the particles may be intermixed with the fibers during deposition - with all options indicated with dotted lines in Fig. 1.

Once the web 100 is formed and inspected, it is transferred to further processing unit(s) 1800, such as cutting and sizing the web into pieces and/or incorporating the web into an article. Either at any of these further downstream process steps, or as a part of the web forming equipment 100, a reject unit 1900 may separate out of specification material, preferably for being recycled.

It is important, that the equipment further comprises a contact free web inspection system 3000, comprising an electromagnetic (hereinafter also referred to as "EM") wave receiver system 3100 adapted to receive electromagnetic waves 160 as pass through or are reflected from the web 100 in the web inspection region 3010. The receiver system 3100comprises at least one, preferably a multiplicity of receiver units 3110 transforming the received EM waves 160 into an electrical signal 170. Preferably, the sensor detecting system should be able to capture the EM wave pictures of the web 100 at a high sampling rate, preferably of at least about 24 kHz or more than about 30 kHz.

Each of the detectors has a predetermined pixel resolution size that is less than about 5 mm by 5 mm, preferably better than 1 mm by 1 mm, more preferably less than 0.3 mm by 0.3 mm or even less than 0.1 by 0.1 mm.

Optionally (and hence indicated with dotted lines in Fig. 1), the system may comprise an emitter for electromagnetic waves 3120, positioned preferably opposite to the receiver, relative to the web 100.

The range of the electromagnetic waves may be from more than about 10 Hz up to about 10¹⁹ Hz.

**Table 1: Wave frequencies**

| Range | Frequency Range | Approximate Wavelength |
|---|---|---|
| Radio Waves | 10¹-10⁹ Hz | >1m |
| Microwaves | 10⁹-10¹² Hz | 1mm-1m |
| Infrared | 10¹²-10¹⁴ Hz | 700nm-1mm |
| Visible Light | 10¹⁴-10¹⁵ Hz | *400-700nm* |
| Ultraviolet | 10¹⁵-10¹⁷ Hz | 10-400nm |
| X-rays | 10¹⁷-10¹⁹ Hz | 0.01-10nm |

In a first particular execution, the wave frequency is in the range of visible light, and conventional video analysis systems can be employed, with suitable systems being available from e.g., Cognex Inc, Natick, MA, USA, or Mitsubishi Electric Europe B.V., Germany, under the trade designation Line Scan Bar. In a second alternative execution, the wave frequency is operated at a range of more than 100 GHz, preferably more than 200 GHz, but less than 900 GHz, preferably less than 600 GHz, most preferably in the range of between 300 GHz and 500 GHz. Suitable detection systems for this execution are disclosed in the above referenced publication WO2018/204724 (Alcatera), to which express reference is made as far as the detection systems are concerned, whilst similar systems are manufactured by Terasense Group Inc. San Jose, CA, USA. In a third alternative execution, the wave frequency is 3x10³ Hz to 3x10⁹ Hz, for which system may be available from Accusentry, Marietta, GA, USA.

The electrical signal 170 is further transmitted via a connection 3190 - by cable or wireless - to a data processing unit 3200, wherein the electrical signal is correlated to at least basis weight and occurrence of nits, clumps and spots of the web by comparing the signal to pre-set target data from a target data storage unit 3210, wherein for various product specifications and raw material properties the target set points for processing units are stored. A further set of data included in this target data storage 3210, or in a separate deviation data storage unit 3215, or calculated from a pre-set algorithm, comprises process reaction parameters as a function of the deviation of the measurement parameter from the target values, from which the corrective action for the various processing units is determined - qualitatively by defining which processing unit control system is actuated, as well as quantitatively by defining the degree of actuation. The data processing unit 3200 may further comprise conventional data processing elements, especially a human interface device 3250 or a data capturing and storage unit 3260 for further evaluation of received data and resulting control actions.

In particular, at least one, preferably two, or more preferably at least three of the control parameters selected from the following group are to be adjusted automatically by the control unit 3200 via the respective connections 3290, as may be via conventional cables, bus architecture, as indicated by connection dots in parts of the connection lines 3290, or also wireless network connections:
a) control of the fiber supply 1110 by control unit 1112 for
   a1) fiber board supply drive 1111 and/or
   a2) fiber bale picker unit 1118;
b) control of the disintegration unit 1200, such as a hammer mill 1210, through disintegration rotational speed controller 1214 for the rotor drive 1213 and/or disintegration gap width 1216 by disintegration gap width control 1217;
c) control of the transfer air supply 1300, especially
   c1) temperature control 1302;
   c2) humidity control 1304;
   c3) air flow volume control 1306;
d) control of the web forming unit 1101, especially for each individual or a group of impellers 1150 by
   d1) control of rotational speed, and/or
   d2) control of the angle of the impeller blades;
e) control of the collector unit 1500, especially
   e1) control of the machine directional speed of the air permeable collector 1527;
   e2) control of main suction fan 1550;
   e3) control of suction for each individual or a group of vacuum suction boxes 1551;
f) control of the reject unit 1900.

In case that such an automatic control cannot control the process parameters towards target web property parameter, the system may provide a signal to an operator and/or induce a shut- down to allow inspection and operator interaction.

The controlling units may comprise stepper motors, pneumatics positioners, or flow diverters. Preferably they comprise closed loop servo motors, exhibiting high dynamics, low response time, preferably of less than 10 ms, and low positioning error. Optionally, they may operate according to pre-set and programmable movement or velocity profiles. Preferably, the PLC units apply a proportional-integral-derivative (PID) control logic, optionally as cascading PID control, e.g., a first level PID controlling deviations resulting from the overall process speed or target web properties, a second level adjusting process parameters according to calculated deviations.

Thus, the computer implemented control method in a continuous manufacturing system for fibrous webs comprises the steps of
A) providing a data processing unit 3200 comprising a data analysis algorithm, a target data storage 3210 and a deviation data storage 3125, further a human interface device 3250 and an output data storage device 3260;
B) receiving in the data processing unit 3200
   a. signals from an EM detection system 3100 and
   b. trigger signals from the collector drive controller 1527;
C) generating an actual deviation profile by comparing the signals from the EM detection system 3100 and the target data storage data 3210 in the data processing system for defined regions of the web;
D) comparing the actual deviation profile to the deviation data storage data 3125 and calculating reaction parameters by predetermined algorithms;
E) sending the reaction parameter of step D) to one or more of
   a) control of the fiber supply 1110 by control unit 1112 for
      a1) fiber board supply drive 1111 and/or
      a2) fiber bale picker unit 1118;
   b) control of the disintegration unit 1200, such as a hammer mill, through disintegration rotational speed controller 1214 for the rotor drive 1213 and/or disintegration gap width 1216 by disintegration gap width control 1217;
   c) control of the transfer air supply 1300, especially
      c1) temperature control 1302;
      c2) humidity control 1304;
      c3) air flow volume control 1306;
   d) control of the web forming unit 1101, especially for each individual or a group of impellers 1150 by
      d1) control of rotational speed, and/or
      d2) control of the angle of the impeller blades;
   e) control of the collector unit 1500, especially
      e1) control of the machine directional speed of the air permeable collector 1527;
      e2) control of main suction fan 1550;
      e3) control of suction for each individual or a group of vacuum suction boxes 1551;
   f) control of reject controller;
F) actuating the respective controller unit according to step E,
whereby steps B to F are essentially continuously executed.

## Claims

1. An equipment (1000) for forming an air-laid web (100) comprising cellulose fibers comprising
1) a short fiber supply unit (1110) for supplying fibrous material comprising cellulosic fibers,
- adapted to provide varying fiber feed rates;
2) a fiber individualizing unit (1200), preferably fiber pick apparatus or a hammer mill,
- adapted to operate at varying individualization degrees;
3) a fiber-in-air suspension unit comprising
3a) a suspension air supply (1300), adapted to adjust
- temperature, water content, air flow rate;
3b) a mixing unit, preferably a venturi unit (1420),
- adapted to suspense said fibrous material in said air;
4) a web forming unit comprising;
4a) a forming box (1101);
4b) a homogenization unit (1150) comprising a multiplicity of impellers, rotatably mounted around z-directional axis, and comprising
4b1) a speed adjustable drive (1158) for one or more of said impellers;
4b2) blades, preferably with adjustable blade angles;
5) a fiber collection unit (1500), comprising
5a) an air permeable collector (1510), preferably a foraminous belt, operating at an adjustable collector speed, adapted for deposition of said fibrous material thereon, thereby forming a web;
5b) a vacuum suction unit (1550), comprising
5b1) an adjustable air blower adapted to be operated at varying air vacuum and volume (150);
5b2) a multiplicity of vacuum suction boxes (1552) connected to said air blower, preferably comprising air flow adjustment baffles;
5b3) optionally a web compaction unit (1610);
5b4) optionally a reject system (1900);
6) a contact free inspection unit (3100)
6a) comprising at least one, preferably a multiplicity of receiver units (3110),
6ai) arranged
- moveable at least cross-directionally or
- in a line or a 2D-matrix;
6aii) adapted to receive electromagnetic (EM) waves from a surface of a web (100) formed on said collector (1510)
6aiii) and adapted to convert EM waves in the range of from 10 Hz to 10¹⁹Hz, preferably in a range selected from the group consisting of
- visible light of about 10¹⁴ to 10¹⁵ Hz
- 10¹¹ to 10¹² Hz;
- 3x10³ Hz to 3x10⁹ Hz
into an electric signal (170);
6b) optionally at least one emitter unit (3120) providing electromagnetic waves with a frequency in the range of from 10 Hz to 10¹⁹Hz to be received by said receiver unit(s);
7) a data processing unit (3200) adapted
- to convert said electric signal (170) of 6a) into an at least x-y-directionally expanding actual property image
preferably with an image resolution of the smaller of
at least three times the size of a receiver unit and
resolution size that is less than about 5 mm by 5 mm, preferably less than 1 mm by 1 mm, more preferably less than 0.3 mm by 0.3 mm or most preferably less than 0.1 by 0.1 mm.
and to compare said actual property image to a target property image and a deviation image,
- and to calculate signals for adjusting at least one of the processing parameters of at least one of said units 1) to 5),
thereby calculating the occurrence of nits, clumps, and spots by changes in the deviation image of more than 30 % of the signal, whereby
nits exhibit a size of equal or less than about 4 mm²;
clumps exhibit a size of more than about 4 mm² and less than about 64 mm²;
spots exhibit a size of more than about 64 mm²;
and wherein each of said units 1 to 5) are adjustable to impact at least one of the web property parameters selected from the group of
- nits occurrence;
- clump occurrence;
- spots occurrence;
- basis weight.

2. An equipment (1000) according to claim 1, wherein said EM waves exhibit a wave frequency in the range of visible light of 10¹⁴ to 10¹⁵ Hz.

3. An equipment (1000) according to claim 1 or 2, wherein said contact free inspection unit and EM wave emitter are positioned at the same side relative to said web.

4. A process for the manufacture of air-laid webs comprising the steps of
a) providing an equipment (1000) according to any of claims 1 to 3;
b) operating said equipment (1000) by
b1) creating a fiber-in-air suspension by individualizing fibers from said fiber supply unit (1110) in an air stream;
b2) feeding said fibers into said web forming unit (1101) and forming a web on said collector (1510) of said fiber collection unit (1500) operating at a collector speed;
c) inspecting said web (100) by said inspection unit (3100) by receiving electromagnetic waves by said receiver unit(s) (3110),
said inspecting comprising the steps of
receiving a time depending signal as a function of the EM wave response parameter as a function of the web property parameters by said receiver unit(s) (3110),
and transmitting said signal to said data processing unit (3200);
d) analysing said signal in said data processing unit (3200) by
- creating a parameter image of the web properties, showing a time dependent CD profile of said web property parameters;
- comparing said parameter image to a pre-determined set of target parameters;
- determining local and time dependent variations of said parameters of said web by synchronizing said parameter image with said collector speed;
e) providing correction signals for any of said units 1) to 5) of said equipment (1000), by comparing said local and time dependent variations of said parameters to a predetermined set of adjustment parameters;
f) adjusting processing parameters of said units 1 to 5),
whereby steps b) to f) are executed continuously.

5. A process according to claim 4, wherein
said forming of a web (100) provides a web (100) with a basis weight of more than about 10 g/m².

6. A process according to claim 4, wherein
said forming of a web (100) provides a web (100) with a basis weight of less than about 200 g/m².

7. A process according to any of claims 4 to 6, wherein said EM waves received by said receiver unit(s) (3110) are in a range selected from the group consisting of
- visible light of about 10¹⁴ to 10¹⁵ Hz;
- 10¹¹ to 10¹² Hz;
- 3x10³ Hz to 3x10⁹ Hz.

8. A computer implemented control method in a manufacturing system for fibrous webs (100) comprising the steps of
A) providing a data processing unit (3200) comprising a data analysis algorithm, a target data storage (3210) and a deviation data storage (3125), further a human interface device (3250) and an output data storage device (3260);
B) receiving in said data processing unit (3200)
a. electrical signals (170) from an EM detection system (3100) and
b. speed related trigger signals from the collector drive controller (1527);
C) generating an actual cross-directional deviation profile by comparing the signals from said EM detection system (3100) and said target data storage data (3210) in the data processing system for defined regions of the web (100);
D) comparing the actual deviation profile to said deviation data storage data (3125) and calculating reaction parameters by predetermined algorithms;
E) sending the reaction parameter of step D) to one or more of
a) control of the fiber supply (1110) by control unit (1112) for
a1) fiber board supply drive (1111) and/or
a2) fiber bale picker unit (1118);
b) control of the disintegration unit (1200), such as a hammer mill, through disintegration rotational speed controller 1214 for the rotor drive 1213 and/or disintegration gap width controller 1217 by disintegration gap width 1216;
c) control of the transfer air supply (1300), especially
c1) temperature control (1302);
c2) humidity control (1304);
c3) air flow volume control (1306);
d) control of the web forming unit (1101), especially for each individual or a group of impellers (1150) by
d1) control of rotational speed, and/or
d2) control of the angle of the impeller blades;
e) control of the collector unit (1500), especially
e1) control of the machine directional speed of the air permeable collector (1527);
e2) control of main suction fan (1550);
e3) control of suction for each individual or a group of vacuum suction boxes (1551);
f) control of the reject actuator (1900);
F) actuating the respective controller unit according to step E,
whereby steps B to F are essentially continuously executed.
